# EUROPEAN PATENT SPECIFICATION

(11) **EP 0 464 120 B1**
(45) Date of publication and mention of the grant of the patent: **22.05.1996**
(21) Application number: 90905579.0
(22) Date of filing: 21.03.1990
(51) Int. Cl.: G01N 33/543, G01N 21/75

(54) **ASSAY METHOD USING SURFACE PLASMON RESONANCE SPECTROMETRY**
VERFAHREN ZUM IMMUNTESTEN UNTER VERWENDUNG DER OBERFLÄCHENPLASMONRESONANZSPEKTROMETRIE
PROCEDE D'ANALYSE UTILISANT LA SPECTROMETRIE PAR RESONANCE DE PLASMONE EN SURFACE

(30) Priority: 23.03.1989 GB 8906776
(43) Date of publication of application: 08.01.1992
(73) Proprietor: AMERSHAM INTERNATIONAL plc, Little Chalfont Buckinghamshire HP7 9NA (GB)
(72) Inventor: CHARLES, Stephen Alexander, Aylesbury, Bucks. HP1 9JB (GB); FINLAN, Martin Francis, Aylesbury, Bucks. HP21 9YU (GB); GARLAND, Peter Bryan, Old Bosham, West Sussex PO18 8HQ (GB); POLLARD-KNIGHT, Denise Vera, Harrow, Middlesex HA1 4BY (GB)
(74) Representative: Pennant, Pyers
(86) International application number: GB9000433
(87) International publication number: WO9011525

(56) References cited:
- EP-A- 0 028 132
- EP-A- 0 276 142
- EP-A- 0 305 109
- ANALYTICAL CHEMISTRY, vol. 56, no. 8, July 1984, American Chemical Society; D. MONROE, pp. 920A-931A/

## Description

This invention concerns methods of assaying for analytes using the technique of surface plasmon resonance spectrometry (SPRS). The method is applicable to analytes generally, but is likely to be of particular interest where the analyte is a hapten (a small molecule capable of being bound by antibody but not of itself immunogenic).

The phenomenon on SPR is well known and will not be described in detail. Two effects are known to give rise to SPR, the Woods effect which involves a metallized diffraction grating; and the Kretchmann effect with which this invention is concerned. Reference is directed to EPA 305109 for a discussion of the Kretchmann effect. Briefly, the intensity of monochromatic plane-polarised light (conveniently obtained from a laser) reflected from the interface between an optically transparent material, e.g. glass, and a thin film of metal depends on the refractive index of material in a thin layer, at most a few hundred nm thick, on the downstream side of the metal. Accordingly, by measuring changes in intensity of reflected light an indication can be obtained of changes in refractive index of material on the metal. The intensity of reflected light also varies with the angle of incidence, and reflectivity drops sharply to a minimum at a particular angle characteristic of the equipment. The metal surface is generally of silver, although this is not critical to the invention.

The immunoassay of haptens by Surface Plasmon Resonance Spectrometry (SPRS) poses a particular problem because the haptens are necessarily of low molecular weight and therefore cause only very small changes in refractive index when they bind to or dissociate from an antibody-coated SPRS silver-coated surface. This problem is addressed in EP O 378 594 (International Application PCT/GB89/00156).

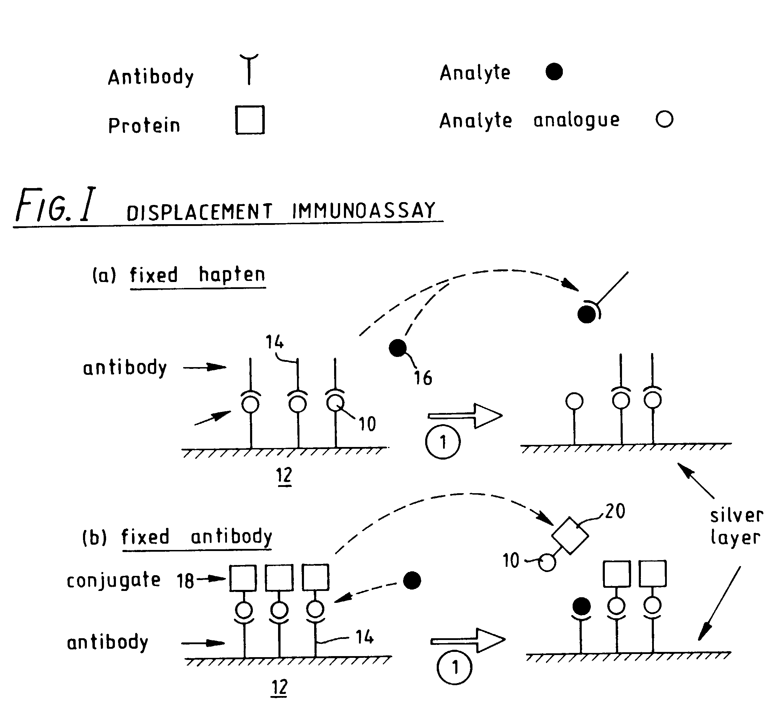

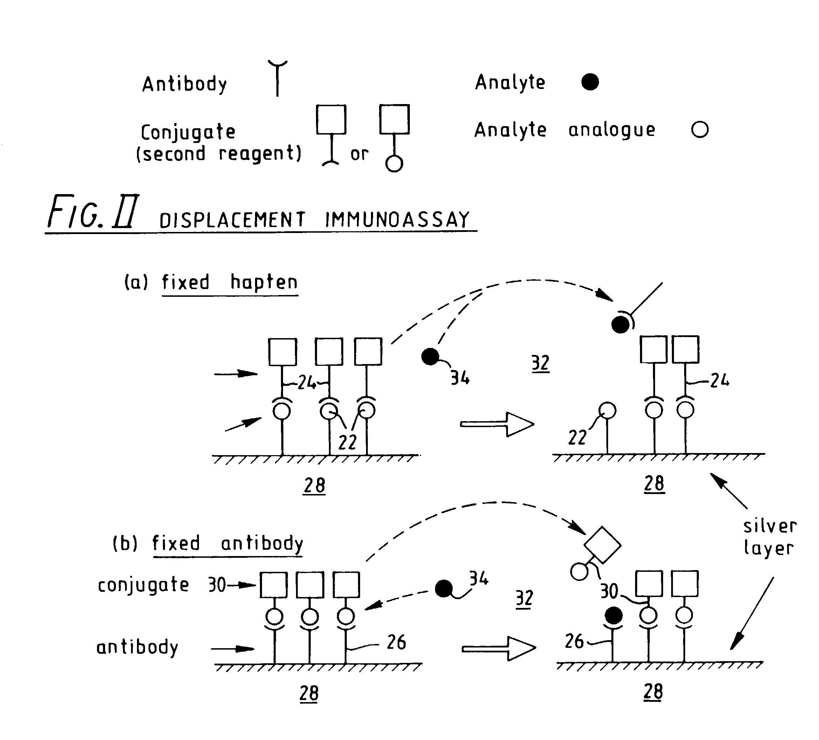

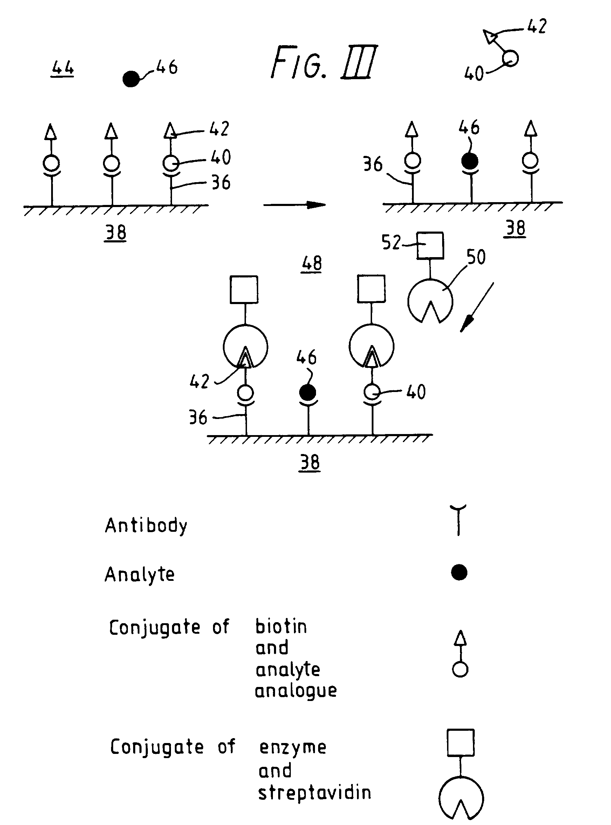

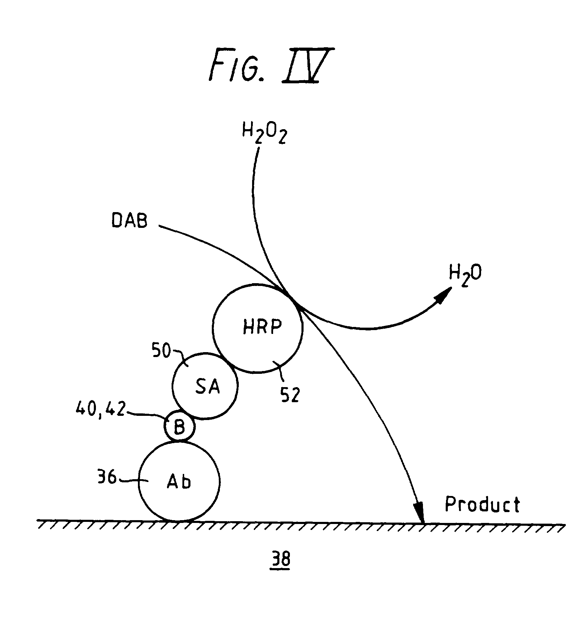

Two ways around this problem are illustrated in Figure 1 of the attached drawings. In 1 (a), the hapten 10 or analogue is immobilised to the silver surface 12 used for SPRS detection, and binds the corresponding antibody 14. Introduction of free hapten 16 (whose concentration it is wished to determine) displaces antibody by competing with surface bound hapten. This displacement of antibody from the surface is detected as an SPRS signal. In 1 (b), the antibody 14 is bound to the surface 12 and binds a conjugate 18 of the hapten 10 or analogue and (typically) a protein 20 of sufficient molecular weight to yield a significant SPRS signal on displacement of the conjugate by added free hapten.

However, this arrangement is not always very sensitive. This invention provides a way of increasing the sensitivity.

EPA 276142 describes an SPR assay in which an assay reagent X is chosen to have a high refractive index so as to enhance an SPR signal. In the system described, deposition of the reagent X is monitored by SPR making use of the Woods effect.

The present invention provides a method of assaying for an analyte which is a member of a specific binding pair, by the use of a solid surface carrying immobilised thereon a first reagent which is a member of the specific binding pair, and of a second reagent which is a conjugate of a member of the specific binding pair, at least one of the first and second reagents being a specific binding partner of the analyte,

wherein there is used as the solid surface a metallic layer applied to a block of material transparent to electromagnetic radiation, the presence of the conjugate on the surface is assayed by surface plasmon resonance spectrometry, and the conjugate is selected to be capable of giving a strong SPR signal,

which method comprises incubating a fluid sample containing the analyte with the first and second reagents, whereby the conjugate is caused to be present on the solid surface in a manner related to the presence of the analyte in the sample.

The invention is characterised in that:
- the second reagent is reversibly bound to the first reagent and the sample is then bought into contact with the solid surface, whereby a proportion of the second reagent, directly related to the concentration of the analyte in the sample, is displaced from the detection surface; or
- the second reagent is a conjugate of a member of the specific binding pair with an enzyme, which enzyme is caused to catalyse a reaction resulting in the production of a reaction product which is deposited on the solid surface.

In one embodiment, either (a) the first reagent is an analogue of the analyte and the second reagent is a conjugate of a specific binding partner of the analyte, or (b) the first reagent is a specific binding partner of the analyte and the second reagent is a conjugate of an analogue of the analyte,
and the assay is performed by effecting competition between the analyte and the analyte analogue for binding with the specific binding partner of the analyte, whereby the conjugate is caused to be present on the solid surface in a manner related to the presence of the analyte in the sample.

An analogue of the analyte is a substance which competes with the analyte for binding to a specific binder therefor. Often the analogue will be arranged to be as near as possible or even completely identical to the analyte. The use in assays of analyte analogues is well known.

In a preferred arrangement, the second reagent is reversibly bound to the first reagent as a preliminary step. When the sample is brought into contact with the solid surface, competition between the analyte and the analyte analogue causes displacement of a proportion of the second reagent from the detection surface, that proportion being directly related to the concentration of the analyte in the sample. Because that second reagent is a conjugate selected to be capable of giving a strong SPR signal, its displacement from the solid surface is readily and accurately monitored by means of SPRS. An advantage of this arrangement is that the assay is performed simply by bringing the sample into contact with the solid surface, no other reagent being required.

Two embodiments of the invention have been designated (a) and (b). in (a), the first reagent immobilised on the solid surface is an analogue of the analyte, preferably the analyte itself carried on a suitable spacer molecule (generally a macromolecule such as a protein). The second reagent is a conjugate of a specific binding partner of the analyte, most usually of an antibody to the analyte.

In (b) the first reagent, immobilised on the solid surface, is a specific binding partner of the analyte, generally an antibody to the analyte. The second reagent is then a conjugate of a the analyte or of an analogue of the analyte.

In one approach, the second reagent is a conjugate with a substance which itself gives a strong SPR signal, as described below.

In another approach, the second reagent is a conjugate with a member of a different specific binding pair which is non-reactive with the analyte. In this case, the assay includes an additional step of bringing into contact with the solid surface a complex of the other member of the different specific binding pair with a substance which itself gives a strong SPR signal. Examples of the different specific pairs include the biotin-avidin and biotin-streptavidin systems. Preferably biotin is present in the second reagent.

This additional step is performed during or after the competition step of the assay. The complex may be present in a fluid which is brought into contact with the solid surface. Or the complex may be incorporated in the fluid sample. For example the fluid sample may be caused to flow successively over two solid surfaces, of which the first carries the complex in soluble form and the second is the SPR detection surface.

The substance may be one having a high refractive index. This may be a molecule or particle with a high refractive index or a large size, to confer a higher refractive index on the reagent as a whole thus giving rise to a larger SPR signal than would be the case with an unmodified molecule. Possible substances include heavy substances (e.g. metal ions or higher halogens) highly electronically delocalised species (e.g. polycyclic aromatics or dyes), metal or metal oxide particles such as titania particles, or high refractive index organic species such as ferritin.

Alternatively, the substance may be one having a low refractive index, i.e. a refractive index lower than that of the environment close to the solid surface.

Alternatively, the substance may be an enzyme which is caused to catalyse a reaction resulting in the production of a reaction product which is deposited on the solid surface. It is necessary that the reaction product resulting from the action of the enzyme be immobilised on or exceedingly close (i.e. within a thin layer of a few hundred nanometers or less) to the metal surface. The reaction product may have a refractive index which is higher or lower than the material present in that thin layer. Examples of suitable enzymes are:-
- a): Peroxidase, with H₂O₂ and diamino-benzidine (DAB) as substrates. The latter is converted to an insoluble product.
- b): Certain oxidoreductases with NAD(P)H and a tetrazolium salt as substrate. The latter is converted to an insoluble product (a formazan). Other reductant and dye combinations exist. (See F P Altmann, 1972, "An introduction to the use of tetrazolium salts in quantitative enzyme cytochemistry" published by Koch-Light Laboratories, Colnbrook, Bucks, England).
- c): Certain catalase enzymes are known to generate gas, bubbles of which can be retained close to the solid surface.

When the second reagent is a conjugate of a member of thespecific binding pair with an enzyme, the assay may conveniently be performed in a sandwich assay format. In this case, both the first and second reagents are specific binding partners of the analyte (i.e. antibodies when the analyte is an antigen or hapten). This is in contrast to the displacement assays described above which are necessarily performed in a competition assay format.

The assay performed by the method may be qualitative, i.e. simply to detect the presence or absence of an analyte in a sample, or quantitative. For quantitative assays, measurements may be made of the rate of change of reflectivity, and/or of the absolute reflectivity at a given time. Contact between the fluid medium and the solid surface may be static, but is more preferably dynamic e.g. by the fluid medium being caused to flow across the metal surface.

The nature of the analyte is not critical. However, for the reasons given above, the invention is of particular value when the analyte is a hapten. The first reagent may be immobilised on the surface of the metal e.g. silver layer by conventional means, e.g. covalent bonding or simply physical adsorption. A biotinylated reagent may be immobilised on a metal surface to which streptavidin has been bound. Indeed immobilisation may involve any binding pair, including DNA/DNA or antibody/antigen.

Reference is directed to Figures II, III and IV of the accompanying drawings which illustrate aspects of the invention, and in which:
Figure II comprises two reaction diagrams illustrating one embodiment;
Figure III is a reaction diagram illustrating another embodiment, and
Figure IV shows the final state of the solid surface when an enzyme is used.
Figure II (a) shows the preliminary step in an assay for an analyte in a sample. At the outset, an analogue 22 of the analyte is irreversibly attached to a silver layer 28, and an antibody conjugate 24 is reversibly bound to the analogue.
Figure II (b) shows a similar arrangement. At the outset, antibody 26 to the analyte is irreversibly attached to the silver layer 28 and an analyte analogue conjugate 30 is reversibly bound to the antibody.

Both systems operate in the same way. On addition of a sample 32 containing the analyte 34, a proportion of the conjugate molecules is displaced from the silver layer, that proportion being directly related to the concentration of the analyte in the sample.

Figure III shows a two-step assay. At the start, antibody 36 to the analyte is irreversibly attached to a silver layer 38, and a conjugate of an analyte analogue 40 with biotin 42 is reversibly bound to the antibody. On addition of a sample 44 containing an analyte 46, a proportion of the conjugate molecules is displaced from the silver layer, that proportion being directly related to the concentration of the analyte in the sample.

A fluid 48 containing a complex of streptavidin 50 with HRP enzyme 52 is then brought into contact with the silver layer. The streptavidin binds to the biotin to an extent inversely related to the concentration of the analyte in the sample.

The final step of the assay is shown in Figure IV. When diamino-benzidine (DAB) and hydrogen peroxide (H₂O₂) are brought into contact with the silver layer, the HRP enzyme catalyses reaction between them leading to formation of an insoluble product which accumulates adjacent the silver layer 38 and gives rise to a strong SPR signal which is easily monitored.

In place of the HRP enzyme 52, there could have been used a high refractive index substance, such as ferritin or a titania particle, as described above.

In another embodiment, an enzyme may be irreversibly bound on the solid surface in an inactive form. When the fluid sample is brought into contact with the solid surface, analyte in the sample may activate the enzyme which may then be used to generate an SPR signal in the manner shown in Figure 4. The enzyme thus activated by the analyte may itself be one which generates an insoluble product; or may be one which generates a substrate for such an enzyme.

The following Examples illustrate the invention

In each of Examples 1 and 2, the analyte is human immunoglobulin, and a sandwich assay format is used. The first reagent, immobilised on a silver surface, is an anti-human immunoglobulin antibody. The second reagent is an (anti-human immunoglobulin antibody) - enzyme conjugate. In a first step, a sample containing the analyte is brought into contact with the silver surface, whereby the analyte binds to the immobilised first reagent (present in excess).

In a second step, a solution of the second reagent is brought into contact with the silver surface, whereby the second reagent becomes bound on the solid surface to an extent proportional to the amount of analyte in the sample. This gives rise to a small but measurable SPRS signal. To amplify the signal, a substrate for the enzyme is supplied to cause an insoluble reaction product to be deposited on the silver surface.

In Examples 1 and 2, the first step is assumed to have taken place, and the second step is performed by applying the second reagent direct to the silver surface. The second step, demonstrated in the Examples, is the key to the method.

### Example 1

An anti-human immunoglobulin-β galactosidase conjugate was diluted to 36nM in 10mN sodium phosphate pH 7.4 and 1ml pumped across a silver slide at 8µl/sec. The shift in the angle at which SPR occurs (change in reflectivity) was monitored on binding the conjugate. 3mls of 10mN sodium phosphate pH7.4 was then manually injected across the slide to wash the surface.

A block of sheep immunoglobulin (2µM, 1ml) in 10mN sodium phosphate pH7.4 3mM NgCl₂, was injected across the slide, followed by a wash of 3mls of 10mN sodium phosphate pH7.4, 3mM NgCl₂, 0.5% BSA. The substrate solution (VLM from PPR Diagnostics, Kings College, London) diluted to 0.4mM in 10mN phosphate buffer pH7.4 was then flowed at 3µl/sec across the slide and the change in reflectivity monitored with time. A wash step of 3mls of 10mN sodium phosphate was then performed.

The results from two experiments are shown in the table below. There is at least a five fold enhancement of the signal on addition of the substrate.

### Example 2

An anti-human immunoglobulin-alkaline phosphatase conjugate diluted to 0.125nM and 60pM in 10mN Tris/HCl pH 9.5, 3mM NgCl₂ (dilution buffer) was flowed across a silver coated slide at 4µl/sec and the change in reflectivity monitored. Following a wash step with 3mls of this same buffer, the slide was blocked with 2µM sheep immunoglobulin in 10mM Tris/HCl pH 9.5, 3mM MgCl₂ (1ml manual injection). A further wash step (3mls of dilution buffer) was performed and the enzyme substrate solution (3.3mg/ml nitro-blue tetrazolium, 1.65mg/ml 5-bromo-4-chloro-3-indoyl-phosphate in dilution buffer) then flowed across the slide at 3µl/sec. The change in reflectivity with time was monitored.

The table below shows the results for 0.125nM and 60pM anti-human immunoglobulin-alkaline phosphatase conjugate respectively. The addition of the enzyme substrate significantly enhances the sensitivity of the assay.

| Final Change in Reflectivity (%) | | |
|---|---|---|
| | Bind of enzyme conjugate | Addition of enzyme substrate |
| Example 1 36nMβ-glactosidase-anti-human immunoglobulin | 7 | 37 |
| Example 2 0.125nM anti-human immunoglobulin-alkaline phosphatase | 4 | 51 |
| 60 pM anti-human immunoglobulin-alkaline phosphate | 2.2 | 28 |

### Example 3

### A Displacement Assay for Human Chorionic Gonadotrophin (HCG) using a Refractive Index Probe

10mM sodium phosphate buffer, pH 7.4 (buffer 1) was pumped across the silver slide and the change in SPR angle monitored. A solution of 166nM human immunoglobulin-HCG conjugate diluted in buffer 1 was then bound to the silver surface by pumping across the surface at 8µl/sec. Following a wash step with buffer 1, the silver surface was then blocked by manual injection of 2µM sheep immunoglobulin diluted in 10mN sodium phosphate buffer, pH7.4 containing 0.5% bovine serum albumin (buffer 2) across the silver slide. A mouse anti-HCG monoclonal antibody, free in solution or linked onto 55nM polystyrene refractive index probes (enhanced sensitivity), was then bound to the previously immobilised HCG-conjugate by pumping a 100nM solution of the anti-HCG antibody diluted in buffer 2 across the silver slide. The silver slide was then washed with buffer 2. 1µM HCG diluted in buffer 2 was then added and displacement of the anti-HCG antibody from the silver surface was observed by SPR. As shown in the table below both the rate of displacement and the final change in the shift in the SPR angle were greater when the anti-HCG antibody was attached to the 55nM polystyrene refractive index probes.

| DISPLACED MOLECULE | | |
|---|---|---|
| SPR Parameter | Anti-HCG antibody immobilised on refractive index probe | Anti-HCG antibody |
| RATE* | -0.0322 | -0.0043 |
| FINAL CHANGE** | -7.4% | -1.2% |

| | | |
|---|---|---|
| * change in reflectivity per second | | |
| ** change in reflectivity at the end of the assay | | |

## Claims

1. A method of assaying for an analyte which is a member of a specific binding pair, by the use of a solid surface carrying immobilised thereon a first reagent which is a member of the specific binding pair, and of a second reagent which is a conjugate of a member of the specific binding pair, at least one of the first and second reagents being a specific binding partner of the analyte,
wherein there is used as the solid surface a metallic layer applied to a block of material transparent to electromagnetic radiation, the presence of the conjugate on the surface is assayed by surface plasmon resonance spectrometry, and the conjugate is selected to be capable of giving a strong SPR signal,
which method comprises incubating a fluid sample containing the analyte with the first and second reagents, whereby the conjugate is caused to be present on the solid surface in a manner related to the presence of the analyte in the sample,
characterised in that the second reagent is reversibly bound to the first reagent and the sample is then brought into contact with the solid surface, whereby a proportion of the second reagent, directly related to the concentration of the analyte in the sample, is displaced from the detection surface.

2. A method of assaying for an analyte which is a member of at least one specific binding pair, by the use of a solid surface carrying immobilised thereon a first reagent which is a member of a said specific binding pair, and of a second reagent which is a conjugate of a member of a said specific binding pair, at least one of the first and second reagents being a specific binding partner of the analyte,
wherein there is used as the solid surface a metallic layer applied to a block of material transparent to electromagnetic radiation, the presence of the conjugate on the surface is assayed by surface plasmon resonance spectrometry, and the conjugate is selected to be capable of giving a strong SPR signal,
which method comprises incubating a fluid sample containing the analyte with the first and second reagents simultaneously or in any desired order, whereby the conjugate is caused to be present on the solid surface in a manner related to the presence of the analyte in the sample,
characterised in that the second reagent is a conjugate of a member of the specific binding pair with an enzyme, which enzyme is caused to catalyse a reaction resulting in the production of a reaction product which is deposited on the solid surface.

3. A method as claimed in Claim 1, wherein the conjugate is with a substance which itself gives a strong SPR signal.

4. A method as claimed in Claim 1, wherein the conjugate is with a member of a different specific binding pair which is non-reactive with the analyte, and the assay includes the additional step of bringing into contact with the solid surface a complex of the other member of the different specific binding pair with the substance which gives a strong SPR signal.

5. A method as claimed in claim 3 or claim 4, wherein the substance is one having a high refractive index.

6. A method as claimed in Claim 2, wherein a sandwich assay format is used.

7. A method as claimed in any one of Claims 1 to 5, wherein either a) the first reagent is an analogue of the analyte and the second reagent is a conjugate of a specific binding partner of the analyte, or b) the first reagent is a specific binding partner of the analyte and the second reagent is a conjugate of an analogue of the analyte, and the incubation step is performed so as to effect competition between the analyte and the analyte analogue for binding with the specific binding partner of the analyte.

8. A method as claimed in any one of Claims 1 to 7, wherein the analyte is a hapten.

## Patentansprüche

1. Verfahren zur Untersuchung bezüglich eines Analyten, bei dem es sich um ein Mitglied eines speziellen Bindungspaars handelt, unter Verwendung einer festen Oberfläche, auf der ein erstes Reagens, bei dem es sich um ein Mitglied des speziellen Bindungspaars handelt, und ein zweites Reagens, bei dem es sich um ein Konjugat eines Mitglieds des speziellen Bindungspaars handelt, immobilisiert gebunden sind, wobei mindestens eines des ersten und zweiten Reagenses ein spezieller Bindungspartner des Analyten ist,
wobei als die feste Oberfläche eine auf einen für elektromagnetische Strahlung durchlässigen Materialblock applizierte Metallschicht verwendet wird, die Anwesenheit des Konjugats auf der Oberfläche durch Oberflächenplasmon-Resonanzspektroskopie untersucht wird und das Konjugat so ausgewählt ist, daß es in der Lage ist, ein starkes SPR-Signal zu liefern,
wobei das Verfahren ein Inkubieren einer flüssigen, den Analyten enthaltenden Probe mit dem ersten und zweiten Reagens umfaßt, wodurch das Konjugat dazu gebracht wird, daß es auf der festen Oberfläche in einer mit der Anwesenheit des Analyten in der Probe im Verhältnis stehenden Weise vorhanden ist,
dadurch gekennzeichnet, daß das zweite Reagens reversibel an das erste Reagens gebunden wird und die Probe anschließend mit der festen Oberfläche in Berührung gebracht wird, wodurch ein Teil des zweiten Reagenses, der im direkten Verhältnis zur Konzentration des Analyten in der Probe steht, von der Nachweisoberfläche verdrängt wird.

2. Verfahren zur Untersuchung bezüglich eines Analyten, bei dem es sich um ein Mitglied mindestens eines speziellen Bindungspaars handelt, unter Verwendung einer festen Oberfläche, auf der ein erstes Reagens, bei dem es sich um ein Mitglied des speziellen Bindungspaars handelt, und ein zweites Reagens, bei dem es sich um ein Konjugat eines Mitglieds des speziellen Bindungspaars handelt, immobilisiert gebunden sind, wobei mindestens eines des ersten und zweiten Reagenses ein spezieller Bindungspartner des Analyten ist,
wobei als die feste Oberfläche eine auf einen für elektromagnetische Strahlung durchlässigen Materialblock applizierte Metallschicht verwendet wird, die Anwesenheit des Konjugats auf der Oberfläche durch Oberflächenplasmon-Resonanzspektroskopie untersucht wird und das Konjugat so ausgewählt ist, daß es in der Lage ist, ein starkes SPR-Signal zu liefern,
wobei das Verfahren ein Inkubieren einer den Analyten enthaltenden, flüssigen Probe mit dem ersten und zweiten Reagens simultan oder in einer beliebigen gewünschten Reihenfolge umfaßt, wodurch das Konjugat dazu gebracht wird, daß es auf der festen Oberfläche in einer mit der Anwesenheit des Analyten in der Probe im Verhältnis stehenden Weise vorhanden ist,
dadurch gekennzeichnet, daß das zweite Reagens ein Konjugat eines Mitglieds des speziellen Bindungspaars mit einem Enzym ist, wobei das Enzym dazu gebracht wird, eine Reaktion zu katalysieren, die zur Bildung eines Reaktionsprodukts führt, das auf der festen Oberfläche abgeschieden wird.

3. Verfahren nach Anspruch 1, wobei das Konjugat ein solches mit einer Substanz ist, die selbst ein starkes SPR-Signal liefert.

4. Verfahren nach Anspruch 1, wobei das Konjugat ein solches mit einem Mitglied eines unterschiedlichen speziellen Bindungspaars ist, das mit dem Analyten keine Reaktion einzugehen vermag, wobei der Assay den weiteren Schritt eines Inkontaktbringens eines Komplexes des anderen Elements des unterschiedlichen speziellen Bindungspaars mit der Substanz, die ein starkes SPR-Signal liefert, mit der festen Oberfläche umfaßt.

5. Verfahren nach Anspruch 3 oder Anspruch 4, wobei die Substanz eine solche mit einem großen Brechungsindex ist.

6. Verfahren nach Anspruch 2, wobei ein Sandwich-Assay-Format verwendet wird.

7. Verfahren nach einem der Ansprüche 1 bis 5, wobei entweder (a) das erste Reagens ein Analoges des Analyten und das zweite Reagens ein Konjugat eines speziellen Bindungspartners des Analyten sind oder (b) das erste Reagens ein spezieller Bindungspartner des Analyten und das zweite Reagens ein Konjugat eines Analogen des Analyten sind und wobei die Inkubationsstufe so erfolgt, daß eine Konkurrenz zwischen dem Analyten und dem Analytenanalogen bezüglich des Bindens an den speziellen Bindungspartner des Analyten durchgeführt wird.

8. Verfahren nach einem der Ansprüche 1 bis 7, wobei der Analyt ein Hapten ist.

## Revendications

1. Procédé d'analyse destiné à un analyte qui est un élément d'une paire spécifique de liaison, en utilisant une surface solide, qui porte, immobilisée sur elle, un premier réactif qui est un élément de la paire spécifique de liaison, et un deuxième réactif qui est un conjugué d'un élément de la paire spécifique de liaison, au moins le premier ou le deuxième réactif étant un partenaire liant spécifique de l'analyte,
dans lequel c'est une couche métallique appliquée sur un bloc de matière transparente à un rayonnement électromagnétique qui est utilisée comme surface solide, la présence du conjugué sur la surface est analysée par spectrométrie par résonnance de plasmon en surface, ou SPR, et le conjugué est sélectionné de manière qu'il soit susceptible de donner un fort signal de SPR,
ce procédé comprenant une incubation d'un échantillon fluide contenant l'analyte avec le premier et le deuxième réactifs, grâce à quoi le conjugué est amené à être présent à la surface solide d'une manière liée à la présence de l'analyte dans l'échantillon,
caractérisé en ce que le deuxième réactif est lié de façon réversible au premier réactif et l'échantillon est ensuite amené au contact de la surface solide, grâce à quoi une certaine proportion du deuxième réactif, directement reliée à la concentration de l'analyte dans l'échantillon, est déplacée de la surface de détection.

2. Procédé d'analyse destiné à un analyte qui est un élément d'au moins une paire spécifique de liaison, en utilisant une surface solide, qui porte, immobilisée sur elle, un premier réactif qui est un élément d'une dite paire spécifique de liaison, et un deuxième réactif qui est un conjugué d'un élément de ladit paire spécifique de liaison, au moins le premier ou le deuxième réactif étant un partenaire liant spécifique de l'analyte,
dans lequel c'est une couche métallique appliquée sur un bloc de matière transparente à un rayonnement électromagnétique qui est utilisée comme surface solide, la présence du conjugué sur la surface est analysée par spectrométrie par résonnance de plasmon en surface, ou SPR, et le conjugué est sélectionné de manière qu'il soit susceptible de donner un fort signal de SPR,
ce procédé comprenant une incubation d'un échantillon fluide contenant l'analyte avec le premier et le deuxième réactifs simultanément ou dans un ordre souhaité quelconque, grâce à quoi le conjugué est amené à être présent à la surface solide d'une manière liée à la présence de l'analyte dans l'échantillon,
caractérisé en ce que le deuxième réactif est un conjugué d'un élément de la paire spécifique de liaison avec un enzyme, enzyme qui est amené à catalyser une réaction dont le résultat est la production d'un produit de réaction qui est déposé sur la surface solide.

3. Procédé selon la revendication 1, dans lequel le conjugué est avec une substance qui donne elle-même un fort signal SPR.

4. Procédé selon la revendication 1 dans lequel le conjugué est avec un élément d'une paire spécifique de liaison différente qui est non réactive avec l'analyte, et l'analyse inclut l'étape additionnelle consistant à amener au contact de la surface solide un complexe de l'autre élément de la paire spécifique de liaison différente avec la substance qui donne un fort signal de SPR.

5. Pprocédé selon la revendication 3 ou la revendication 4, dans lequel la substance est une substance à indice de réfraction élevé.

6. Procédé selon la revendication 2, dans lequel est utilisé un format d'analyse en sandwich.

7. Procédé selon l'une quelconque des revendications 1 à 5 dans lequel, soit a) le premier réactif est un analogue de l'analyte et le second réactif est un conjugé d'un partenaire liant spécifique de l'analyte, soit b) le premier réactif est un partenaire liant spécifique de l'analyte et le deuxième réactif est un conjugué d'un analogue de l'analyte, et l'étape d'incubation est effectuée de manière à effectuer une compétition, entre l'analyte et l'analogue de l'analyte, pour la liaison avec le partenaire liant spécifique de l'analyte.

8. Procédé selon l'une quelconque des revendications 1 à 7, dans lequel l'analyte est un haptène.
